# EUROPÄISCHE PATENTANMELDUNG

(11) **EP 1 064 955 A1**
(43) Veröffentlichungstag der Anmeldung: **03.01.2001**
(21) Anmeldenummer: 00113553.2
(22) Anmeldetag: 27.06.2000
(51) Int. Cl.: A61L 2/18

(54) **Fusssprüheinrichtung**

(30) Priorität: 02.07.1999 DE 19930613
(71) Anmelder: Friedrich Grohe AG & Co. KG, 58675 Hemer (DE)
(72) Erfinder: Luig, Frank-Thomas, 58708 Menden (DE); Philipps-Liebich, Hartwig, 58675 Hemer (DE)

(57) **Zusammenfassung**

Bei einer Fusssprüheinrichtung mit einer Einrichtung zum dosierten Abgeben von Desinfektionsflüssigkeit über wenigstens eine Sprühdüse, ist zur Verbesserung vorgeschlagen, dass ein Annäherungsschalter zur berührungslosen Einleitung der Abgabe der Desinfektionsflüssigkeit vorgesehen ist.

## Beschreibung

Die Erfindung betrifft eine Fusssprüheinrichtung, insbesondere für den Einsatz in Badeanlagen, mit einer Einrichtung zum dosierten Abgeben von Desinfektionsflüssigkeit über wenigstens eine Sprühdüse.
Es sind Fusssprüheinrichtungen bekannt, bei denen der Austritt der Desinfektionsflüssigkeit an der Sprühdüse über ein mechanisches Selbstschlussventil gesteuert wird, wobei die Laufzeit des Selbstschlussventils einstellbar ist. Die Desinfektionsflüssigkeit wird hierbei meist durch Zudosieren von Desinfektionsmittelkonzentrat zu aus dem Trinkwasserleitungsnetz entnommenem Druckwasser in der Einrichtung selbst hergestellt und vom Selbstschlussventil dosiert über eine oder mehrere Sprühdüsen abgegeben. Zur Einleitung des Sprühvorgangs ist es erforderlich, dass der Benutzer das Selbstschlussventil manuell betätigt.

Der Erfindung liegt die Aufgabe zugrunde, die bekannte Fusssprüheinrichtung zu verbessern, wobei insbesondere eine Verbesserung des Einschalt- oder Auslösevorgangs erzielt werden soll.

Diese Aufgabe wird erfindungsgemäss dadurch gelöst, dass ein Annäherungsschalter zur berührungslosen Einleitung der Abgabe der Desinfektionsflüssigkeit vorgesehen ist.
Weitere Ausgestaltungen der Erfindung sind in den Ansprüchen 2 bis 11 angegeben.

Mit den vorgeschlagenen Massnahmen wird insbesondere erreicht, dass die Desinfektionsflüssigkeit ohne manuelle Betätigung in der gewünschten Menge dargeboten wird, wodurch eine erhebliche Steigerung des Benutzungskomforts erreicht wird.
Ausserdem kann mit der vorgesehenen elektrischen Steuerung, vorzugsweise einer Steuerung mit einem Mikroprozessor, der Austrittszeitraum, in dem Desinfektionsflüssigkeit versprüht wird, zeitgenau eingestellt werden, so dass der Verbrauch an Desinfektionsflüssigkeit optimiert werden kann. Darüber hinaus kann durch die Zusammenfassung der Sprühbrause mit der Abdeckrosette und der Erfassungssensorik zu einer Funktionseinheit eine vereinfachte Installationsmontage und eine erleichterte Pflege und Reinigung ermöglicht werden.

Ein Ausführungsbeispiel ist in der Zeichnung dargestellt und wird im folgenden näher beschrieben. Es zeigt
- Fig. 1: einen Teil zweier, durch eine Gebäudewand getrennte Räume mit einer Fusssprüheinrichtung in schematischer Darstellung;
- Fig. 2: einen Teil der in Fig. 1 gezeigten Fusssprüheinrichtung im Längsschnitt in vergrösserter Darstellung.

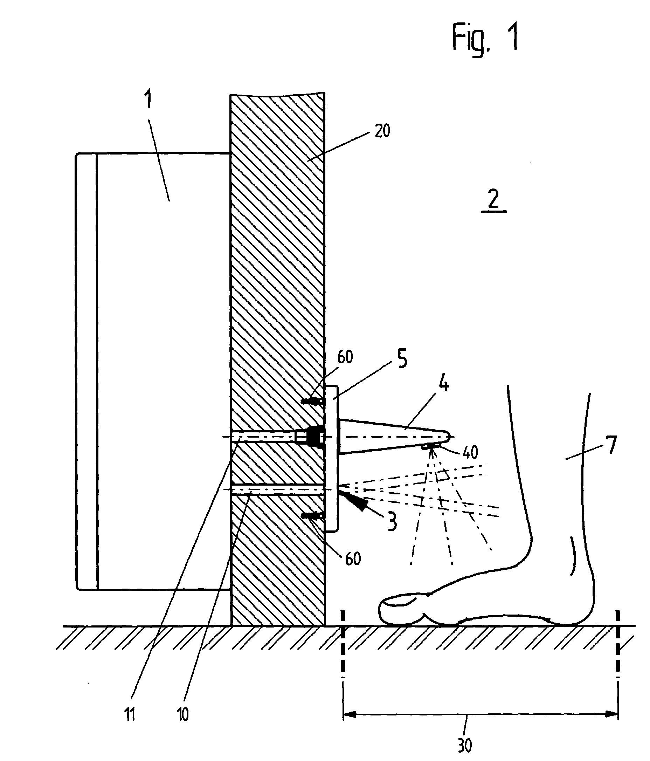

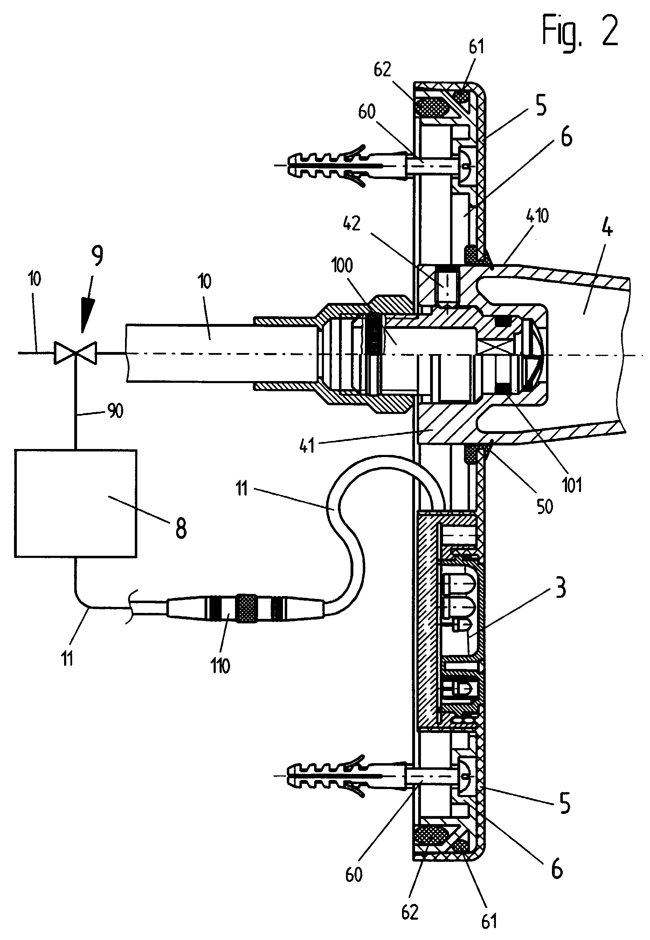

In Fig. 1 ist ein Teil eines Raums 2, insbesondere eines Wasch- oder Duschraums, dargestellt, der von einer Gebäudewand 20 begrenzt ist, wobei an der Aussenseite der Gebäudewand 20, ausserhalb des Raums 2, eine Einrichtung zur dosierten Abgabe von Desinfektionsflüssigkeit in schematischer Weise dargestellt ist. Von der Einrichtung 1 ist eine Leitung 10 für die Zuführung der Desinfektionsflüssigkeit zu einer an der Gebäudewand 20 befestigten, in den Raum 2 sich erstreckenden Sprühbrause 4 vorgesehen. Daneben ist eine Leitung 11 für eine elektrische Verbindung zwischen einer, in der Einrichtung 1 angeordneten elektrischen Steuerung 8 und einer Erfassungssensorik 3 in einer, an einer Gebäudewand befestigbaren und auf der Sprühbrause 4 aufgeschobenen Abdeckrosette 5 angeordnet. Wie es insbesondere aus Fig. 2 der Zeichnung zu entnehmen ist, ist im Verlauf der Leitung 10 ein Magnetventil 9 vorgesehen, welches mit einer Ansteuerleitung 90 mit der als Blockschaltbild dargestellten elektrischen Steuerung 8 verbunden ist. Zur einfachen Installation ist ausserdem in der Leitung 11 eine Verbindungskupplung 110 vorgesehen.

Die Erfassungssensorik 3 ist in der Abdeckrosette 5 integriert, wobei die Abdeckrosette 5 in der Montageposition mit einem Dichtring 50 auf einer zylindrischen Mantelfläche 410 am stromaufwärts gelegenen Endbereich der Sprühbrause 4 axial verschiebbar aufgenommen ist. Zur dichten Anordnung der Abdeckrosette 5 an der Gebäudewand 20 ist ausserdem ein Tragkörper 6 vorgesehen, der mittels Dübelschrauben 60 an der Gebäudewand 20 fixiert werden kann. Hierbei ist in einer umlaufenden Nut ein Dichtring 62 vorgesehen, mit dem der Tragkörper 60 zur Gebäudewand 20 abgedichtet wird. Ausserdem ist im Aussenmantelbereich in einer umlaufenden Nut ein weiterer Dichtring 61 angeordnet, mit dem eine Abdichtung zwischen dem Tragkörper 6 und der Innenwandung des umlaufenden Mantels der Abdeckrosette 5 erfolgt. Die Sprühbrause 4 mit der Abdeckung, bestehend aus der Abdeckrosette 5 und dem Tragkörper 6, und der Erfassungssensorik 3 bilden somit eine Funktionseinheit.

An der Leitung 10 ist ein Anschlussnippel 100 angeordnet, an dessen zylindrischer Mantelfläche ein Dichtring 101 positioniert ist. Die Sprühbrause 4 ist dabei mit einem Anschlussteil 41 versehen, welcher auf den Anschlussnippel 100 aufgesteckt werden kann, wobei der Dichtring 101 die Abdichtung zwischen dein Anschlussnippel 100 und dem Anschlussteil 41 der Sprühbrause 4 herstellt. Zur Sicherung der Sprühbrause 4 in der Stecklage ist ferner eine Radialschraube 42 in dem Anschlussteil 41 vorgesehen, die gegen die Mantelfläche des Anschlussnippels 100 verspannt werden kann.

Die Fusssprüheinrichtung hat folgende Funktionsweise:
In der Einrichtung 1 wird Desinfektionsflüssigkeit mit einem Wasserleitungsdruck von etwa 3 bar bereitgestellt. Von der Erfassungssensorik 3 wird Infrarotlicht abgestrahlt. Die Erfassungssensorik 3 ist hierbei so ausgelegt, dass sie Reflektionen, verursacht durch einen Benutzer 7 in einem bestimmten Erfassungsbereich 30, empfängt und ein entsprechendes Signal an die Steuerung 8 abgibt. Hierauf wird von der Steuerung 8 ein Ablaufprogramm initiiert, worauf das Magnetventil 9 für einen bestimmten Zeitraum geöffnet wird. Während der Öffnungszeit tritt Desinfektionsflüssigkeit über die Sprühbrause 4 an der Sprühdüse 40 aus, so dass die Füsse des Benutzers 7 entsprechend desinfiziert werden können. Die Parameter für das Ablaufprogramm können beispielsweise über Potentiometer oder mit Infrarotsignalen, die von einer Fernsteuerungseinheit erzeugt und von der Erfassungssensorik 3 empfangen werden, eingestellt werden.

Alternativ kann die Steuerung 8 auch so ausgebildet sein, dass das Magnetventil 9 während des gesamten Zeitraums, in dem von der Erfassungssensorik 3 ein Benutzer 7 im Erfassungsbereich 30 detektiert wird, in Offenstellung verharrt.

Bei dem vorstehend beschriebenen Ausführungsbeispiel arbeitet die Erfassungsensorik mit Infrarotlicht. Selbstverständlich können aber auch Annäherungsschalter mit beispielsweise einem Ultraschall-, Radar- oder Kapazitivsensor vorgesehen werden.

## Patentansprüche

1. Fusssprüheinrichtung, insbesondere für den Einsatz in Badeanlagen, mit einer Einrichtung zum dosierten Abgeben von Desinfektionsflüssigkeit über wenigstens eine Sprühdüse, dadurch gekennzeichnet, dass ein Annäherungsschalter zur berührungslosen Einleitung der Abgabe der Desinfektionsflüssigkeit vorgesehen ist.

2. Fusssprüheinrichtung nach Anspruch 1, dadurch gekennzeichnet, dass der Annäherungsschalter mit einer elektrischen Steuerung (8) verbunden ist, die die Sprühdauer bestimmt.

3. Fusssprüheinrichtung nach Anspruch 1, dadurch gekennzeichnet, dass die Sprühdauer von der Anwesenheitsdauer eines Benutzers (7) im Erfassungsbereich (30) des Annäherungsschalters bestimmt ist.

4. Fusssprüheinrichtung nach einem der Ansprüche 1 bis 3, dadurch gekennzeichnet, dass als Annäherungsschalter ein Infrarot-, Ultraschall-, Radar- oder Kapazitivsensor vorgesehen ist.

5. Fusssprüheinrichtung nach wenigstens einem der Ansprüche 1 bis 4, dadurch gekennzeichnet, dass die Einrichtung (1) zur dosierten Abgabe der Desinfektionsflüssigkeit verdeckt angeordnet und mit wenigstens einer Leitung (10) in einen Raum (2) eines Gebäudes mit einer Sprühdüse (40) hineingeführt ist, wobei an der Gebäudewand (20) des Raums (2) im Bereich der Leitung (10) eine Abdeckrosette (5) angeordnet ist, in der eine Erfassungssensorik (3) des Annäherungsschalters integriert ist.

6. Fusssprüheinrichtung nach Anspruch 5, dadurch gekennzeichnet, dass die Einrichtung (1) hinter der Gebäudewand (20) angeordnet ist und entsprechende Leitungen (10,11) durch die Gebäudewand (20) zu einer Sprühbrause (4) und der Erfassungssensorik (3) vorgesehen sind.

7. Fusssprüheinrichtung nach Anspruch 6, dadurch gekennzeichnet, dass die Leitung (10) für die Zuführung der Desinfektionsflüssigkeit einen Anschlussnippel (100) mit einem Dichtring (101) trägt, auf dem die Sprühbrause (4) mit einem Anschlussteil (41) aufgesteckt ist, wobei das Anschlussteil (41) am Endbereich eine zylindrische Mantelfläche (410) hat, auf der die Abdeckrosette (5) mit einem Dichtring (50) in der Stecklage aufgenommen ist.

8. Fusssprüheinrichtung nach einem der Ansprüche 5 bis 7, dadurch gekennzeichnet, dass die Abdeckrosette (5) von einem an der Gebäudewand (20) befestigbaren Tragkörper (6) in der Stecklage gehalten ist.

9. Fusssprüheinrichtung nach Anspruch 8, dadurch gekennzeichnet, dass der Tragkörper (6) mittels Dübelschrauben (60) an der Gebäudewand (20) befestigt ist.

10. Fusssprüheinrichtung nach Anspruch 9, dadurch gekennzeichnet, dass der Tragkörper (6) einen Dichtring (62) zur Abdichtung zur Gebäudewand (20) und einen weiteren Dichtring (61) zur Abdichtung zur Abdeckrosette (5) trägt.

11. Fusssprüheinrichtung nach wenigstens einem der Ansprüche 1 bis 10, dadurch gekennzeichnet, dass die Sprühbrause (4) mit der Abdeckung, bestehend aus der Abdeckrosette (5) und dem Tragkörper (6), und der Erfassungssensorik (3) zu einer Funktionseinheit zusammengefasst sind.
